# EUROPEAN PATENT APPLICATION

(11) **EP 4 755 290 A1**
(43) Date of publication of application: **10.06.2026**
(21) Application number: 24217646.9
(22) Date of filing: 05.12.2024
(51) Int. Cl.: A61B 5/00, A61B 5/024, A61B 5/1455, A61B 1/07, A61B 5/0295

(54) **OPTICAL MEASUREMENT DEVICE**

(71) Applicant: Koninklijke Philips N.V., 5656 AE Eindhoven (NL)
(72) Inventor: PAULUSSEN, Elvira Johanna Maria, Eindhoven (NL); KOSTAKIS, Dimitri George, Eindhoven (NL)
(74) Representative: Philips Intellectual Property & Standards

(57) **Abstract**

An optical measurement device (100) for obtaining optical measurements from a tissue region (110), is provided. The optical measurement device includes an optical source (120); an optical detector (130); a first optical waveguide (140); and a second optical waveguide (150). In one option, i) a proximal end portion (140_{Pep}) of the first optical waveguide adjoining the proximal end (140') of the first optical waveguide, and at least a portion of the optical source (120), are in direct contact with a first material (160) having a refractive index (n₁) that is less than a refractive index (n₃) of the first optical waveguide (140), and also less than a refractive index (n_{S}) of the optical source (120), for containing the optical radiation within the first optical waveguide (140) and/or within the optical source (120), respectively. In another option, ii) a proximal end portion (150_{Pep}) of the second optical waveguide adjoining the proximal end (150') of the second optical waveguide, and at least a portion of the optical detector (130), are in direct contact with a first material (160) having a refractive index (n₁) that is less than a refractive index (n₃) of the second optical waveguide (150), and also less than a refractive index (n_{D}) of the optical detector (130), for containing the optical radiation within the second optical waveguide (150) and/or within the optical detector (130), respectively.

## Description

### FIELD OF THE INVENTION

The present disclosure relates to an optical measurement device for obtaining optical measurements from a tissue region. The optical measurement device may be used to obtain optical measurements such as photoplethysmography "PPG" measurements, laser speckle plethysmography "SPG" measurements, and so forth. The optical measurement device may be used to obtain optical measurements from tissue regions such as the finger, the ear, the nasal alar, the nasal septum, and so forth. The optical measurements may be used to evaluate a vital signs measurement for a subject, such as the subject's heart rate, temperature, blood oxygen saturation, e.g. SPO₂, and so forth.

### BACKGROUND OF THE INVENTION

Optical measurement devices are often used to obtain optical measurements from tissue. For instance, optical measurements devices are often used to obtain PPG measurements, i.e. measurements of blood volume changes in tissue. Optical measurements may be obtained from tissue in various anatomical locations. For instance, PPG measurements may be obtained from the finger, the ear, the nasal alar, the nasal septum, and so forth. Such PPG measurements may be used to evaluate a vitals signs measurement for the subject, e.g. an SPOz measurement.

Optical measurement devices typically employ one or more optical sources and one or more corresponding optical detectors. The optical source(s) generate optical radiation that is used to irradiate the tissue. The optical detector(s) detect optical radiation that is collected from the tissue in response to the irradiation of the tissue. Electrical signals generated by the optical detector(s) in response in response to the detected optical radiation are indicative of the optical measurements. The optical source(s) and the corresponding optical detector(s) are sometimes arranged in a side-by-side configuration in order to obtain a reflective optical measurement from the tissue. Alternatively, the optical source(s) and optical detector(s) may be arranged in an opposing configuration in order to obtain a transmissive optical measurement from the tissue.

Some types of optical measurement devices obtain their optical measurements by placing their optical source(s) and their optical detector(s) in direct contact with tissue. Such arrangements can provide high optical coupling efficiency with the tissue. Other types of optical measurement devices include one or more optical waveguide(s) between their optical sources(s) and the tissue, or between their optical detector(s) and the tissue. In these arrangements, the optical waveguide(s) couple optical radiation between the optical source(s) and the tissue, and between the tissue and the optical detector(s). The use of such optical waveguide(s) can increase the freedom with which the optical source(s) and the optical detector(s) can be positioned with respect to the tissue. For instance, it may enable the optical source(s) and the optical detector(s) to be positioned further away from the tissue. The use of such optical waveguide(s) can also obviate the need to locate electrical wires close to the tissue that might otherwise be needed to couple electrical signals to and from the optical source(s) and the optical detector(s). Both of these factors enable optical measurements to be made on tissue in environments such as X-ray, and also magnetic resonance imaging "MRI", imaging environments, and in which the presence of electrical wires in the proximity of the tissue presents problems.

However, the assembly of optical measurement devices that employ optical waveguides is challenging. For instance, it can be challenging to accurately align the optical waveguide(s) with their respective optical source(s) and optical detector(s). Accurate alignment of these components is necessary in order to maximize the optical coupling efficiency between the waveguide(s) and their respective optical source(s) and optical detector(s). The alignment of these components is difficult due to their small size. One solution to this problem is to combine multiple optical waveguides into a so-called bundle. The bundle may be a bundle of single optical fibers, for example. The bundle has a larger cross sectional area than that of its individual optical fibers. This relaxes the alignment accuracy that is required between the bundle and the corresponding optical source(s) or optical detector(s). However, a bundle is less flexible than a single optical fiber.

Thus, there is a need to improve the optical coupling efficiency between optical sources/ detectors and their corresponding optical waveguide(s) in optical measurement devices.

### SUMMARY OF THE INVENTION

The invention is defined by the claims.

According to one aspect of the present disclosure, an optical measurement device for obtaining optical measurements from a tissue region, is provided. The optical measurement device includes:
an optical source;
an optical detector;
a first optical waveguide; and
a second optical waveguide.

The first optical waveguide is configured to couple optical radiation generated by the optical source, from a proximal end of the first optical waveguide to a distal end of the first optical waveguide, for irradiating the tissue region when the tissue region is in optical communication with the distal end of the optical waveguide.

The second optical waveguide is configured to couple at least a portion of the optical radiation collected from the tissue region in response to the irradiating, from a distal end of the second optical waveguide to the optical detector, via a proximal end of the second optical waveguide.

Moreover: i) a proximal end portion of the first optical waveguide adjoining the proximal end of the first optical waveguide, and at least a portion of the optical source, are in direct contact with a first material having a refractive index n₁ that is less than a refractive index n₃ of the first optical waveguide, and also less than a refractive index n_{S} of the optical source, for containing the optical radiation within the first optical waveguide and/or within the optical source, respectively;
and/or
ii) a proximal end portion of the second optical waveguide adjoining the proximal end of the second optical waveguide, and at least a portion of the optical detector, are in direct contact with a first material having a refractive index n₁ that is less than a refractive index n₃ of the second optical waveguide, and also less than a refractive index n_{D} of the optical detector, for containing the optical radiation within the second optical waveguide and/or within the optical detector, respectively.

In the above optical measurement device, the optical coupling efficiency between the optical source and the first optical waveguide is improved by providing that i) a proximal end portion of the first optical waveguide adjoining the proximal end of the first optical waveguide, and at least a portion of the optical source, are in direct contact with a first material having a refractive index n₁ that is less than a refractive index n₃ of the first optical waveguide, and also less than a refractive index n_{S} of the optical source. This is because the direct contact, in combination with these relative refractive index values, contains the optical radiation within the first optical waveguide and/or within the optical source, respectively, via total internal reflection. In turn, this relaxes the alignment tolerance that is required between the optical source and the first optical waveguide, thereby simplifying the assembly of the optical measurement device.

In the above optical measurement device, the optical coupling efficiency between the optical detector and the second optical waveguide is improved by providing that ii) a proximal end portion of the second optical waveguide adjoining the proximal end of the second optical waveguide, and at least a portion of the optical detector, are in direct contact with a first material having a refractive index n₁ that is less than a refractive index n₃ of the second optical waveguide, and also less than a refractive index n_{D} of the optical detector. This is because the direct contact, in combination with these relative refractive index values, contains the optical radiation within the second optical waveguide and/or within the optical detector, respectively, via total internal reflection. In turn, this relaxes the alignment tolerance that is required between the optical detector and the second optical waveguide, thereby simplifying the assembly of the optical measurement device.

According to one aspect of the present disclosure, i) a proximal end portion of the first optical waveguide adjoining the proximal end of the first optical waveguide, and at least a portion of the optical source, are in direct contact with a first material having a refractive index n₁ that is less than a refractive index n₃ of the first optical waveguide, and also less than a refractive index n_{S} of the optical source, for containing the optical radiation within the first optical waveguide and/or within the optical source, respectively. At least a portion of the first material, and a further portion of the first optical waveguide adjoining the proximal end portion of the first optical waveguide, are in direct contact with a second material having a refractive index n₂ that is less than a refractive index n₃ of the first optical waveguide for containing the optical radiation within the first optical waveguide.

In this aspect, the optical coupling efficiency between the optical source and the first optical waveguide is further improved by providing the second material having a refractive index n₂ that is less than a refractive index n₃ of the first optical waveguide. This is because the direct contact, in combination with these relative refractive index values, contains the optical radiation within the first optical waveguide, via total internal reflection. In turn, this relaxes the alignment tolerance that is required between the optical source and the first optical waveguide, thereby simplifying the assembly of the optical measurement device. The value of the refractive index n₂ may be approximately equal to the value of the refractive index n₁.

According to one aspect of the present disclosure, ii) a proximal end portion of the second optical waveguide adjoining the proximal end of the second optical waveguide, and at least a portion of the optical detector, are in direct contact with a first material having a refractive index n₁ that is less than a refractive index n₃ of the second optical waveguide, and also less than a refractive index n_{D} of the optical detector, for containing the optical radiation within the second optical waveguide and/or within the optical detector, respectively. At least a portion of the first material, and a further portion of the second optical waveguide adjoining the proximal end portion of the second optical waveguide, are in direct contact with a second material having a refractive index n₂ that is less than a refractive index n₃ of the second optical waveguide for containing the optical radiation within the second optical waveguide.

In this aspect, the optical coupling efficiency between the optical detector and the second optical waveguide is further improved by providing the second material having a refractive index n₂ that is less than a refractive index n₃ of the second optical waveguide. This is because the direct contact, in combination with these relative refractive index values, contains the optical radiation within the second optical waveguide, via total internal reflection. In turn, this relaxes the alignment tolerance that is required between the optical detector and the second optical waveguide, thereby simplifying the assembly of the optical measurement device. The value of the refractive index n₂ may be approximately equal to the value of the refractive index n₁.

According to one aspect of the present disclosure, at least a portion of the second material is formed in an elongate shape having a longitudinal extent. The at least a portion of the second material is configured to contain the optical radiation within the first optical waveguide along the longitudinal extent of the elongate shape.

In this aspect, the second material contains the optical radiation within the first optical waveguide along the longitudinal extent of the elongate shape. The second material may be said to act as a cladding of the first optical waveguide. This simplifies the assembly of the optical measurement device. Moreover, it enables optical measurements to be made in a tissue region that is remote from the optical source, e.g. in an MRI or X-ray imaging environment.

According to one aspect of the present disclosure, at least a portion of the second material is formed in an elongate shape having a longitudinal extent. The at least a portion of the second material is configured to contain the optical radiation within the second optical waveguide along the longitudinal extent of the elongate shape.

In this aspect, the second material contains the optical radiation within the second optical waveguide along the longitudinal extent of the elongate shape. The second material may be said to act as a cladding of the second optical waveguide. This simplifies the assembly of the optical measurement device. Moreover, it enables optical measurements to be made in a tissue region that is remote from the optical detector, e.g. in an MRI or X-ray imaging environment.

According to one aspect of the present disclosure, the further portion of the first optical waveguide extends between the proximal end portion of the first optical waveguide and the distal end of the first optical waveguide. The second material comprises a tissue interface surface for interfacing with the tissue region. The distal end of the first optical waveguide terminates at the tissue interface surface for irradiating the tissue region via the distal end of the first optical waveguide.

In this aspect, a tissue interface surface is provided by the second material. The second material therefore serves the dual purpose of interfacing with the tissue, as well as containing optical radiation within the optical waveguide. This facilitates a more straightforward assembly of the optical measurement device.

According to one aspect of the present disclosure, the tissue interface surface comprises at least one of: an absorbing region, a specular reflecting region, and a diffuse reflecting region. The absorbing region, or the specular reflecting region, or the diffuse reflecting region, is disposed between the distal end of the first optical waveguide and the distal end of the second optical waveguide. In this position, the absorbing region has the effect of reducing the opportunity for optical radiation to couple between the distal end of the first optical waveguide and the distal end of the second optical waveguide without having passed through the tissue region. Such optical radiation might otherwise propagate between the distal end of the first optical waveguide and the distal end of the second optical waveguide via multiple reflections between the tissue interface surface and the surface of the tissue region. This optical radiation typically does not include useful information relating to biological effects within the tissue region, e.g. measurements of blood volume changes in the tissue region, and simply results in an offset in the electrical signal that is generated by the optical detector. In this position, the specular reflecting region, and the diffuse reflecting region, have the effect of re-cycling optical radiation that escapes from the tissue region, instead returning it to the tissue region, where it may acquire information relating to biological effects within the bulk of the tissue region, and ultimately propagate within the tissue region (e.g. via optical scattering) until it is incident on the distal end of the second optical waveguide, and ultimately be detected by the optical detector. This improves the signal-to-noise ratio of the electrical signal that is generated by the optical detector. A combination of an absorbing region, and one or more specular/ diffuse reflecting regions may also be used. For instance, an absorbing region may be arranged in the form of a barrier between the distal end of the first optical waveguide and the distal end of the second optical waveguide, and the specular/ diffuse reflecting region(s) may be arranged around the distal end(s) of the first and/or second optical waveguide(s). This avoids the offset in the electrical signal that is generated by the optical detector, and also improves its signal-to-noise ratio.

According to one aspect of the present disclosure, the further portion of the second optical waveguide extends between the proximal end portion of the second optical waveguide and the distal end of the second optical waveguide. The second material comprises a tissue interface surface for interfacing with the tissue region. The distal end of the second optical waveguide terminates at the tissue interface surface for collecting the at least a portion of the optical radiation from the tissue region via the distal end of the second optical waveguide.

In this aspect, a tissue interface surface is provided by the second material. The second material therefore serves the dual purpose of interfacing with the tissue, as well as containing optical radiation within the second optical waveguide. This facilitates a more straightforward assembly of the optical measurement device.

According to one aspect of the present disclosure, a cross sectional area of the distal end of the first optical waveguide is relatively larger than a cross sectional area of the proximal end of the first optical waveguide. At least a portion of the first optical waveguide between the distal end of the first optical waveguide and the proximal end of the first optical waveguide comprises a tapered cross sectional area.

In this aspect, the tapered cross sectional area of the first optical waveguide increases the effective area of the optical source at the distal end of the first optical waveguide. This improves the optical coupling efficiency between the optical source and the tissue region.

According to one aspect of the present disclosure, a cross sectional area of the distal end of the second optical waveguide is relatively larger than a cross sectional area of the proximal end of the second optical waveguide. At least a portion of the second optical waveguide between the distal end of the second optical waveguide and the proximal end of the second optical waveguide comprises a tapered cross sectional area.

In this aspect, the tapered cross sectional area of the second optical waveguide increases the effective area of the optical detector at the distal end of the second optical waveguide. This improves the optical collection efficiency of the optical radiation from the tissue region.

According to one aspect of the present disclosure, the distal end of the first optical waveguide and/or the distal end of the second optical waveguide comprises a curved profile. The curved profile defines a convex lens.

In this aspect, the convex lens controls the spatial distribution of the optical radiation in the tissue region after it leaves the first optical waveguide and/or controls the volume of the tissue region from which optical radiation is collected by the second optical waveguide. Both, in-turn, improve the optical collection efficiency of the second optical waveguide.

According to one aspect of the present disclosure, the distal end of the first optical waveguide and the distal end of the second optical waveguide are arranged in a side-by-side configuration for obtaining a reflective optical measurement from the tissue region.

In this aspect, the arrangement of the distal ends of the optical waveguides facilitates a reflective optical measurement to be obtained from the tissue region.

According to one aspect of the present disclosure, the distal end of the first optical waveguide and the distal end of the second optical waveguide are arranged in an opposing configuration for obtaining a transmissive optical measurement from the tissue region.

In this aspect, the arrangement of the distal ends of the optical waveguides facilitates a transmissive optical measurement to be obtained from the tissue region.

According to one aspect of the present disclosure, the first material comprises a rigid material and/or the second material comprises a rigid or a flexible material.

In this aspect, the rigid material enables a robust optical connection to be made between the optical source and the first optical waveguide, or between the optical detector and the second optical waveguide, respectively. The flexible material provides a flexible path along the path of respective optical wave guide (s).

According to one aspect of the present disclosure, the first material and/or the second material comprises at least one of: a transparent material, an opaque material, a scattering material, and an absorbing material. The use of opaque, scattering, or absorbing materials prevents the further propagation of any optical radiation that does leak-out into the first/ second material, e.g. optical radiation that does not satisfy the conditions for total internal reflection in the material contacting the first/ second material.

According to one aspect of the present disclosure, the optical source and the optical detector are provided by a photonic integrated circuit, "PIC".

In this aspect, the use of a PIC facilitates a more straightforward assembly of the optical measurement device.

According to one aspect of the present disclosure, the PIC further comprises at least a second optical source. The first optical waveguide is further configured to couple optical radiation generated by the at least second optical source, into the first optical waveguide.

In this aspect, the first optical waveguide is used to couple optical radiation from both optical sources to the tissue region. This provides a more straightforward assembly of the optical measurement device than e.g. using separate optical waveguides to couple optical radiation from the two sources to the tissue region.

According to one aspect of the present disclosure, an optical measurement system is provided. The optical measurement system may be used to obtain optical measurements from a tissue region. The optical measurement system includes:
the optical measurement device described according to any above aspect; and
a controller;

The controller is configured to:
generate control signals for causing the optical source to generate the optical radiation; receive electrical signals generated by the optical detector in response to the detection of the at least a portion of the optical radiation;
evaluate a vital signs measurement based on the received electrical signals; and
output the vital signs measurement.

In this aspect, the optical measurement system is used to evaluate a vital signs measurement. Thus, the optical measurement system may be used to assess the health of a subject.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating a first example of an optical measurement device 100, in accordance with some aspects of the present disclosure.
Fig. 2 is a schematic diagram illustrating a distal end portion of a second optical measurement device 200, in accordance with some aspects of the present disclosure.
Fig. 3 is a schematic diagram illustrating a third example of an optical measurement device 300, in accordance with some aspects of the present disclosure.
Fig. 4 is a schematic diagram illustrating a fourth example of an optical measurement device 400, in accordance with some aspects of the present disclosure.
Fig. 5, which is a schematic diagram illustrating a fifth example of an optical measurement device 500, in accordance with some aspects of the present disclosure.
Fig. 6 is a schematic diagram illustrating a sixth example of an optical measurement device 600, in accordance with some aspects of the present disclosure.
Fig. 7 is a schematic diagram illustrating an example of a portion of a photonic integrated circuit 190 including two optical sources 120₁, and 120₂, in accordance with some aspects of the present disclosure.
Fig. 8 is a schematic diagram illustrating an example of an optical measurement system 700, in accordance with some aspects of the present disclosure.

### DETAILED DESCRIPTION OF EMBODIMENTS

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to one optical measurement device, may be implemented in another optical measurement device, or in an optical measurement system, in a corresponding manner.

In the following description, reference is made to examples of an optical measurement device for obtaining optical measurements from a tissue region. In some examples, reference is made to the optical measurement device being used to obtain PPG measurements from a finger. In these examples, the PPG measurements serve as an example of optical measurements, and the finger serves as an example of a tissue region. It is, however, to be appreciated that unless explicitly stated, the use of the optical measurement device is not limited to these examples. The optical measurement device may be used to make a wide range of optical measurements. For instance, instead of PPG measurements, the optical measurement device may be used to make SPG measurements. It is also to be appreciated that the optical measurement device may be used to make optical measurements from tissue regions other than a finger. The optical measurement device may be used to make optical measurements from tissue in general. For instance, instead of being used to make optical measurements from the finger, the optical measurement device may be used to make optical measurements from the ear, the nasal alar, the nasal septum, and so forth.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the system disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there is a need to improve the optical coupling efficiency between optical sources/ detectors and their corresponding optical waveguide(s) in optical measurement devices.

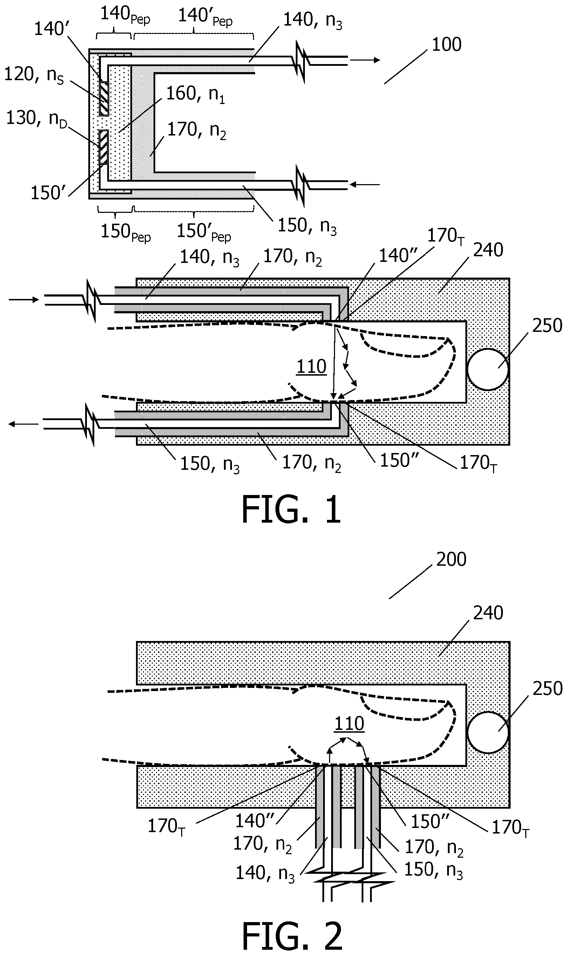

Fig. 1 is a schematic diagram illustrating a first example of an optical measurement device 100, in accordance with some aspects of the present disclosure. The optical measurement device 100 includes an optical source 120. The optical source 120 may be provided by a variety of different optical emitters, including for example light emitting diodes "LEDs", e.g. Superluminescent diodes "SLDs"; lasers, e.g. Vertical-cavity surface-emitting lasers "VCSELS"; incandescent lamps, and so forth. The optical source may generate optical radiation within one or more wavelength intervals. For instance, the optical source may generate optical radiation within a wavelength interval that corresponds to the red portion of the optical spectrum (e.g. approximately 660 nm/ 525 nm), within a wavelength interval that corresponds to the green portion of the optical spectrum, within a wavelength interval that corresponds to the infrared portion of the optical spectrum (e.g. approximately 880 nm or 940 nm, respectively). Such wavelength intervals are often used to perform PPG measurements such as blood oxygen saturation measurements. It is noted that whilst the optical measurement device 100 illustrated in Fig. 1 is illustrated as having a single optical source 120, the optical measurement device 100 may alternatively include multiple optical sources. Where additional source(s) are included, these may be provided in a similar manner.

The optical measurement device 100 illustrated in Fig. 1 also includes an optical detector 130. The optical detector 130 may be provided by a variety of different types of optical detectors, including for example photodiodes, photodetectors, and so forth. The optical detector 130 is sensitive to optical radiation within the one or more wavelength intervals that are generated by the optical source 120. It is noted that whilst the optical measurement device 100 illustrated in Fig. 1 is illustrated as having a single optical detector 130, the optical measurement device 100 may alternatively include multiple optical detectors. Where additional detector(s) are included, these may be provided in a similar manner.

The optical source 120 and the optical detector 130 may be provided in various forms. For instance, the optical source and the optical detector may be provided so-called die form. A single die may include one or more emitters, or one or more detectors. The optical source 120 and the optical detector 130 may also be coupled to a common substrate (not illustrated in Fig. 1). The optical source 120 and the optical detector 130 may alternatively be provided by a photonic integrated circuit, "PIC". The first and/or second optical waveguides 140, 150, may also be provided by the PIC. A PIC is generally defined as a microchip that includes two or more photonic components that form a functioning circuit. The use of a PIC offers advantages such as miniaturization, simplified assembly, inherent component alignment, reduced cost, and so forth.

With continued reference to Fig. 1, the optical measurement device 100 includes a first optical waveguide 140 and a second optical waveguide 150. The first optical waveguide 140 is configured to couple optical radiation generated by the optical source 120, from a proximal end 140' of the first optical waveguide to a distal end 140" of the first optical waveguide, for irradiating the tissue region 110 when the tissue region is in optical communication with the distal end 140" of the first optical waveguide 140. The second optical waveguide 150 is configured to couple at least a portion of the optical radiation collected from the tissue region 110 in response to the irradiating, from a distal end 150" of the second optical waveguide to the optical detector 130, via a proximal end 150' of the second optical waveguide.

In the example illustrated in Fig. 1, the first and second optical waveguides 140, 150, are provided by a so-called fiber waveguide. A fiber waveguide is provided by a fiber of material such as glass (e.g. silica), or a polymer. The fiber defines an elongate path for optical radiation. As illustrated in Fig. 1, the fiber waveguide may have an elongate shape in order to facilitate the irradiation of tissue regions that are distant from the optical sources. Instead of being provided by a fiber waveguide, the first and second optical waveguides 140, 150, may alternatively be provided in other geometric forms, including in the form of a planar waveguide, or a strip waveguide. In these forms, the first and second optical waveguides 140, 150, may likewise be formed from various materials, e.g. silicon-based materials, polymers, and so forth.

In the example illustrated in Fig. 1, the proximal end 140' of the first optical waveguide 140 is butt-coupled to the optical source 120 in order to couple optical radiation that is generated by the optical source 120, into the proximal end 140' of the first optical waveguide 140. Similarly, the proximal end 150' of the second optical waveguide 150 is butt-coupled to the optical detector 130 in order to couple the at least a portion of the optical radiation that has been collected from the tissue region 110 in response to the irradiating, into the optical detector 130.

It is noted that the optical coupling efficiency of the butt-coupled arrangements described above may be further improved by index-matching, or by using an antireflection coating. For instance, the optical coupling efficiency between the optical source 120 and the first optical waveguide 140, and the optical coupling efficiency between the optical detector 130 and the second optical waveguide 150, may be improved by arranging that the refractive index, n₃, of the optical waveguide is approximately equal to, i.e. within approximately ±10% of, the refractive index of the corresponding optical source 120 or optical detector 130. This reduces the Fresnel reflection at these interfaces. Alternatively, or additionally, an antireflection coating may be disposed on the optical waveguide, or on the corresponding optical source 120/ optical detector 130.

Instead of butt coupling the proximal ends 140', 150', of the first and second optical waveguides 140, 150 to the source 120, and to the detector 130, respectively, alternative coupling techniques may be used. For example, one or more of the following may be used: a grating coupling; a refractive, diffractive, a resonant, lens; a metalens; a gradient index "GRIN" lens; and evanescent coupling. Examples of these, and other, coupling techniques are described in a document by Yu, S., et al., "Optical Free-Form Couplers for High-density Integrated Photonics (OFFCHIP): A Universal Optical Interface". February 2020, Journal of Lightwave Technology PP(99):1-1.

In the example illustrated in Fig. 1, distal end 140" of the first optical waveguide and the distal end 150" of the second optical waveguide are arranged in an opposing configuration for obtaining a transmissive optical measurement from the tissue region 110. An alternative arrangement is illustrated in Fig. 2, which is a schematic diagram illustrating a distal end portion of a second optical measurement device 200, in accordance with some aspects of the present disclosure. In the example illustrated in Fig. 2, the distal end 140" of the first optical waveguide and the distal end 150" of the second optical waveguide are arranged in a side-by-side configuration for obtaining a reflective optical measurement from the tissue region 110. It is noted that only the distal end portion of the optical measurement device 200 is illustrated in Fig. 1; the proximal end portion in this example being the same as that illustrated in Fig. 1. It is noted that features illustrated in Fig. 2 that share the same label as Fig. 1 serve the same function as that described with reference to Fig. 1 and that a description of their function is omitted for brevity.

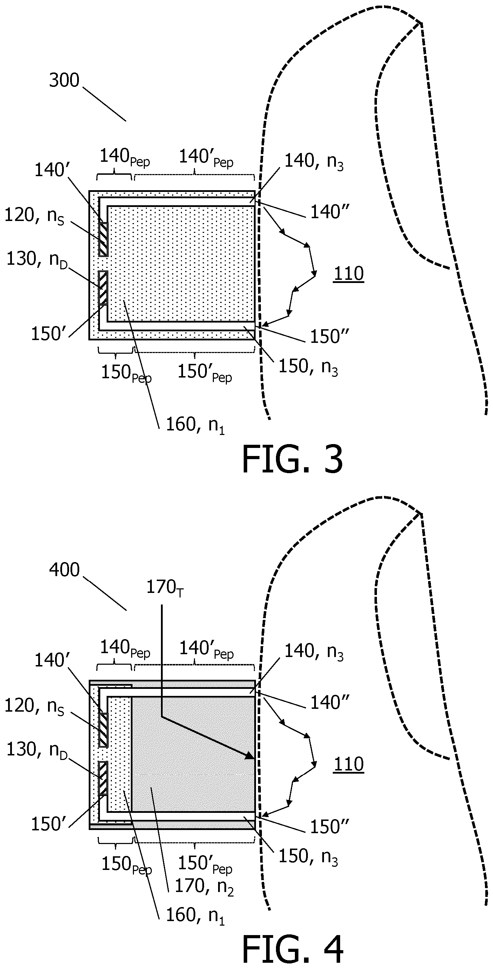

Yet further arrangements in which the distal end 140" of the first optical waveguide and the distal end 150" of the second optical waveguide are arranged in a side-by-side configuration for obtaining a reflective optical measurement from the tissue region 110, are illustrated in Fig. 3 - Fig. 6. For instance, Fig. 3 is a schematic diagram illustrating a third example of an optical measurement device 300, in accordance with some aspects of the present disclosure. It is noted that features illustrated in Fig. 3 - Fig. 6 that share the same label as Fig. 1 serve the same function as that described with reference to Fig. 1 and that a description of their function is omitted for brevity. The arrangements illustrated in Fig. 3 - Fig. 6 differ from the arrangements illustrated in Fig. 1 and in Fig. 2 in that the first and second optical waveguides are relatively shorter than those illustrated in Fig. 1 and in Fig. 2. The arrangements illustrated in Fig. 3 - Fig. 6 are therefore more compact. Thus, the arrangement illustrated in Fig. 3 - Fig. 6 may be used to obtain optical tissue measurements from a tissue region that is closer to the optical source 120 and the optical detector 130.

In the examples illustrated in Fig. 1 - Fig. 6, the tissue region 110 is a portion of a finger. Examples of optical paths within the tissue regions 110 in these examples are illustrated via arrows. For example, in the arrangement illustrated in Fig. 1, optical transmission is measured and this has a direct transmission component (left-hand optical path in Fig. 1), and also a scattering component (right-hand path in Fig. 1). In the arrangements illustrated in Fig. 2 - Fig. 6, optical reflectance is measured and this has a scattering component and no direct transmission measurement. This type of reflectance measurement may be referred-to as a diffuse reflectance measurement. In other arrangements, reflectance measurements may be obtained with both a scattering component and non-negligible direct transmission measurement.

In the examples illustrated in Fig. 1 and Fig. 2, the distal end 140" of the first optical waveguide and the distal end 150" of the second optical waveguide are supported by a mount 240. The mount 240 serves to interface the distal end 140" of the first optical waveguide and the distal end 150" of the second optical waveguide with the tissue region 110. In the examples illustrated in Fig. 1 and Fig. 2, the mount 240 is provided in the form of a clamp. The clamp is provided in two parts that are coupled via a hinge 250. The hinge 250 includes a spring that forces the two parts of the mount together such that when a finger is inserted between the two parts of the mount, the spring applies pressure to the mount, thereby interfacing the distal ends of the first and second optical waveguides with the finger. The clamp 240 provides a robust optical interface with the tissue region 110. The example illustrated in Fig. 3 may be integrated into a mount that is similar to that illustrated in Fig. 1 and Fig. 2.

It is noted that instead of supporting the distal ends of the optical waveguides using the mount 240 illustrated in Fig. 1 and Fig. 2, alternative types of mounts may be used. For instance, the mount 240 may alternatively be provided in the form of a sleeve for receiving a finger; or in the form of a clamp for clamping onto the ear, the nasal alar, or onto the nasal septum; or in the form of a strap for coupling to a limb; or in the form of the body of a wristwatch for coupling to the wrist. Such mounts may support the distal ends of their optical waveguides in an opposing arrangement, or in a side-by-side arrangement, to likewise obtain transmissive, or reflective, optical measurements from a tissue region.

With continued reference to the example illustrated in Fig. 1, in one example, i) a proximal end portion 140_{Pep} of the first optical waveguide adjoining the proximal end 140' of the first optical waveguide, and at least a portion of the optical source 120, are in direct contact with a first material 160 having a refractive index n₁ that is less than a refractive index n₃ of the first optical waveguide 140, and also less than a refractive index n_{S} of the optical source 120, for containing the optical radiation within the first optical waveguide 140 and/or within the optical source 120, respectively.

This is illustrated in the upper portion of Fig. 1, and wherein the first material 160 is in direct contact with both the proximal end portion 140_{Pep} of the first optical waveguide and also the optical source 120. In other words, the first material 160 touches the proximal end portion 140_{Pep} of the first optical waveguide and also the optical source 120.

In this example, the optical coupling efficiency between the optical source and the first optical waveguide is improved by providing that i) a proximal end portion of the first optical waveguide adjoining the proximal end of the first optical waveguide, and at least a portion of the optical source, are in direct contact with a first material having a refractive index n₁ that is less than a refractive index n₃ of the first optical waveguide, and also less than a refractive index n_{S} of the optical source. This is because the direct contact, in combination with these relative refractive index values, contains the optical radiation within the first optical waveguide and/or within the optical source, respectively, via total internal reflection. In turn, this relaxes the alignment tolerance that is required between the optical source and the first optical waveguide, thereby simplifying the assembly of the optical measurement device.

In the example illustrated in Fig. 1, a cross sectional dimension (e.g. area) of the proximal end portion of the first optical waveguide is approximately equal to a cross sectional dimension (e.g. area) of the optical source. In other arrangements, the optical coupling efficiency between the optical source and the first optical waveguide may be increased by arranging that a cross sectional dimension (e.g. area) of the proximal end portion of the first optical waveguide is greater than a cross sectional dimension (e.g. area) of the optical source.

In another example, the first material 160 is used to achieve a similar effect in the optical detector 130 and in the second optical waveguide 150. In this example (which may be used in combination with the above-described example i)), ii) a proximal end portion 150_{Pep} of the second optical waveguide adjoining the proximal end 150' of the second optical waveguide, and at least a portion of the optical detector 130, are in direct contact with a first material 160 having a refractive index n₁ that is less than a refractive index n₃ of the second optical waveguide 150, and also less than a refractive index n_{D} of the optical detector 130, for containing the optical radiation within the second optical waveguide 150 and/or within the optical detector 130, respectively.

This is illustrated in the upper portion of Fig. 1, and wherein the first material 160 is in direct contact with both the proximal end portion 150_{Pep} of the second optical waveguide and also the optical detector 130. In other words, the first material 160 touches the proximal end portion 150_{Pep} of the second optical waveguide and also the optical detector 130.

In this example, the optical coupling efficiency between the optical detector and the second optical waveguide is improved by providing that ii) a proximal end portion of the second optical waveguide adjoining the proximal end of the second optical waveguide, and at least a portion of the optical detector, are in direct contact with a first material having a refractive index n₁ that is less than a refractive index n₃ of the second optical waveguide, and also less than a refractive index n_{D} of the optical detector. This is because the direct contact, in combination with these relative refractive index values, contains the optical radiation within the second optical waveguide and/or within the optical detector, respectively, via total internal reflection. In turn, this relaxes the alignment tolerance that is required between the optical detector and the second optical waveguide, thereby simplifying the assembly of the optical measurement device.

In the example illustrated in Fig. 1, a cross sectional dimension (e.g. area) of the proximal end portion of the second optical waveguide is approximately equal to a cross sectional dimension (e.g. area) of the optical detector. In other arrangements, the optical coupling efficiency between the optical detector and the second optical waveguide may be increased by arranging that a cross sectional dimension (e.g. area) of the proximal end portion of the second optical waveguide is greater than a cross sectional dimension (e.g. area) of the optical detector.

In the examples described above, the first material 160 may be provided in various forms. For instance, the first material 160 may be provided in the form of a drop, blob, molded shape, or as a (deposited) layer. The positioning requirement of the first material 160, and also its shape, is relatively relaxed, and so the first material 160 may be applied using techniques such as printing (e.g. injection from a nozzle), molding, and layer deposition. The first material 160 may be provided by various materials. The first material 160 may be provided by a polymer (e.g. silicone), or by a polymer resin for example. The first material 160 may comprise a rigid material. Using a rigid material enables a robust optical connection to be made between the optical source 120 and the first optical waveguide 140, or between the optical detector 130 and the second optical waveguide 150, respectively. Examples of suitable rigid materials include Polycarbonate, Poly(methyl methacrylate) "PMMA"; Cyclic olefin copolymer "COC"; Cyclic olefin polymers "COP"; Polyethylene terephthalate "PET". Using a rigid material provides a robust connection. The first material 160 may include at least one of: a transparent material, an opaque material, a scattering material, and an absorbing material. The use of opaque, scattering, or absorbing materials prevents the further propagation of any optical radiation that does leak-out into the first material, e.g. optical radiation that does not satisfy the conditions for total internal reflection in the material contacting the first material.

In the arrangements described under i) above, at least a portion of the optical source 120 is in direct contact with the first material 160. In some examples, the at least a portion of the optical source 120 includes a surface of a radiation-emitting region of the optical source. Some types of optical sources (e.g. LEDs and lasers) generate optical radiation within a so-called active region; also referred-to as an active layer. In some examples of these types of optical sources, optical radiation is emitted directly from the active region of the optical source. In these examples, the radiation-emitting region of the optical source is provided by the active region. Thus, in these examples, the at least a portion of the optical source 120 that is in direct contact with the first material includes the surface of the active region of the optical source. In these examples, the first material has the effect of containing optical radiation within the optical source, or more specifically within the active region, via total internal reflection. In other examples of these types of optical sources, optical radiation is likewise generated within an active region, but the optical radiation then travels via a further optical interface region (e.g. a waveguide that forms part of the optical source, a doped contact region, a doped injection region, and so forth) as it leaves the optical source. In these examples, the radiation-emitting region of the optical source is provided by the optical interface region. Thus, in these examples, the at least a portion of the optical source 120 that is in direct contact with the first material includes the surface of the optical interface region. In these examples, the first material has a similar effect of containing optical radiation within the optical source via total internal reflection. In other examples of the arrangements described under i) above, the at least a portion of the optical source 120 includes a surface of another region of the optical source, i.e. a region of the optical source that does not emit radiation. In these examples the at least a portion of the optical source 120 may include e.g. a cladding region, or a metallization region of the optical source. In these arrangements, the first material does not contain optical radiation within the optical source, and instead provides a mechanical connection between the first optical waveguide 140 and the optical source 120.

In the arrangements described under ii) above, at least a portion of the optical detector 130 is in direct contact with the first material 160. In some examples, the at least a portion of the optical detector 130 includes a surface of a radiation-receiving region of the optical detector. As with the optical sources described above, some types of optical detectors (e.g. junction photodiodes) detect optical radiation within a so-called radiation-sensitive region. In some examples of these types of optical detectors, optical radiation is received directly in the radiation-sensitive region of the optical detector, i.e. without traveling via a further optical interface region (e.g. a waveguide that forms part of the optical detector, a doped contact region, a doped injection region, and so forth). In these examples, the radiation-receiving region of the optical detector is provided by the radiation-sensitive region. Thus, in these examples, the at least a portion of the optical detector 130 that is in direct contact with the first material includes the surface of the radiation-sensitive region of the optical detector. Thus, in these examples, the first material has the effect of containing optical radiation within the optical detector, or more-specifically within the radiation-sensitive region, via total internal reflection. In other examples of these types of optical detectors, optical radiation is likewise detected within a radiation-sensitive region, but the optical radiation travels via a further optical interface region (e.g. a doped contact region, a doped injection region, and so forth) prior to being detected by the radiation-sensitive region. In these examples, the radiation-receiving region of the optical source is provided by the optical interface region. Thus, in these examples, the at least a portion of the optical source 120 that is in direct contact with the first material includes the surface of the optical interface region. In these examples, the first material has a similar effect of containing optical radiation within the optical detector via total internal reflection. In other examples of the arrangements described under ii) above, the at least a portion of the optical detector 130 includes a surface of another region of the optical detector, i.e. a region of the optical detector that does not receive radiation. In these examples the at least a portion of the optical detector 130 may include e.g. a metallization region of the optical detector. In these arrangements, the first material does not contain optical radiation within the optical detector, and instead it provides a mechanical connection between the second optical waveguide 150 and the optical detector 130.

In the arrangements described under i) above, the refractive index n_{S} of the optical source 120 refers to the refractive index of the radiation-emitting region of the optical source; e.g. to the active region of the optical source in optical sources that emit optical radiation directly from the active region, or to the refractive index of the optical interface region of the optical source in optical sources that emit optical radiation via a further optical interface.

In the arrangements described under ii) above, the refractive index n_{D} of the optical detector 130 refers to the refractive index of the radiation-receiving region of the optical detector; e.g. to the radiation-sensitive region of the optical detector in optical detectors that receive optical radiation directly in a radiation-sensitive region, or to the refractive index of the optical interface region of the optical detector in optical detectors that receive optical radiation via a further optical interface.

In some examples, the optical measurement device described above is provided with a second material 170. In these examples, at least a portion of the first material 160, and a further portion of the first or second optical waveguide adjoining the proximal end portion of the respective waveguide, are in direct contact with the second material 170. The second material is illustrated in Fig. 1, however, its inclusion is entirely optional.

In one of these examples, i) a proximal end portion 140_{PeP} of the first optical waveguide adjoining the proximal end 140' of the first optical waveguide, and at least a portion of the optical source 120, are in direct contact with a first material 160 having a refractive index n₁ that is less than a refractive index n₃ of the first optical waveguide 140, and also less than a refractive index n_{S} of the optical source 120, for containing the optical radiation within the first optical waveguide 140 and/or within the optical source 120, respectively. In this example, at least a portion of the first material 160, and a further portion 140'_{Pep} of the first optical waveguide adjoining the proximal end portion 140_{PeP} of the first optical waveguide, are in direct contact with a second material 170 having a refractive index n₂ that is less than a refractive index n₃ of the first optical waveguide 140 for containing the optical radiation within the first optical waveguide 140.

In this example, the optical coupling efficiency between the optical source and the first optical waveguide is further improved by providing the second material having a refractive index n₂ that is less than a refractive index n₃ of the first optical waveguide. This is because the direct contact, in combination with these relative refractive index values, contains the optical radiation within the first optical waveguide, via total internal reflection. In turn, this relaxes the alignment tolerance that is required between the optical source and the first optical waveguide, thereby simplifying the assembly of the optical measurement device.

This example is described with reference to Fig. 1, and wherein the second material 170 is shown. As illustrated in the upper portion of Fig. 1, the second material 170 is in direct contact with at least a portion of the first material 160. In other words, the second material 170 touches the at least a portion of the first material 160. The second material 170 is also in direct contact with, i.e. touches, the further portion 140'_{Pep} of the first optical waveguide that adjoins the proximal end portion 140_{PeP} of the first optical waveguide. The second material 170 may be said to provide a cladding for the further portion 140'_{Pep} of the first optical waveguide.

The second material 170 in the above example may be provided in various forms, as described above in relation to the first material 160. The second material 170 may be provided by various materials, as described above in relation to the first material. In one example, the second material comprises a flexible material. Using a flexible material provides a flexible path along the path of respective optical waveguide(s). In one example, the second material is provided by a silicone material. Other examples of suitable flexible materials include polyurethane, e.g. thermoplastic polyurethane "TPU"; or Polyethylene. In one example, the second material comprises a rigid material. Examples of suitable rigid materials include Polycarbonate, Poly(methyl methacrylate) "PMMA"; Cyclic olefin copolymer "COC"; Cyclic olefin polymers "COP"; Polyethylene terephthalate "PET". Using a rigid material provides a robust connection. The second material 170 may include at least one of: a transparent material, an opaque material, a scattering material, and an absorbing material. The use of opaque, scattering, or absorbing materials prevents the further propagation of any optical radiation that does leak-out into the second material, e.g. optical radiation that does not satisfy the conditions for total internal reflection in the material contacting the second material. The value of the refractive index n₂ may be approximately equal to the value of the refractive index n₁.

The second material 170 may similarly be used in combination with the second optical waveguide. Thus, in another of these examples, ii) a proximal end portion 150_{Pep} of the second optical waveguide adjoining the proximal end 150' of the second optical waveguide, and at least a portion of the optical detector 130, are in direct contact with a first material 160 having a refractive index n₁ that is less than a refractive index n₃ of the second optical waveguide 150, and also less than a refractive index n_{D} of the optical detector 130, for containing the optical radiation within the second optical waveguide 150 and/or within the optical detector 130, respectively. In this example, at least a portion of the first material 160, and a further portion 150'_{Pep} of the second optical waveguide adjoining the proximal end portion 150_{Pep} of the second optical waveguide, are in direct contact with a second material 170 having a refractive index n₂ that is less than a refractive index n₃ of the second optical waveguide 150 for containing the optical radiation within the second optical waveguide 150.

In this example, the optical coupling efficiency between the optical detector and the second optical waveguide is further improved by providing the second material having a refractive index n₂ that is less than a refractive index n₃ of the second optical waveguide. This is because the direct contact, in combination with these relative refractive index values, contains the optical radiation within the second optical waveguide, via total internal reflection. In turn, this relaxes the alignment tolerance that is required between the optical detector and the second optical waveguide, thereby simplifying the assembly of the optical measurement device.

This example is described with reference to Fig. 1, and wherein the second material 170 is shown. As illustrated in the upper portion of Fig. 1, the second material 170 is in direct contact with at least a portion of the first material 160. In other words, the second material 170 touches the at least a portion of the first material 160. The second material 170 is also in direct contact with, i.e. touches, the further portion 150'_{Pep} of the second optical waveguide that adjoins the proximal end portion 150_{Pep} of the second optical waveguide. The second material 170 may be said to provide a cladding for the further portion 140'_{Pep} of the first optical waveguide.

The second material 170 in the above example may be provided in various forms, as described above in relation to the first material 160. The second material 170 may be provided by various materials, as described above in relation to the first material. In one example, the second material is provided by a silicone material. In one example, the second material comprises a flexible material. Using a flexible material provides a flexible path along the path of respective optical waveguide(s). The value of the refractive index n₂ of the second material may be approximately equal to the value of the refractive index n₁.

In one example, the at least a portion of the second material 170 is formed in an elongate shape having a longitudinal extent. The at least a portion of the second material 170 is configured to contain the optical radiation within the first optical waveguide 140 along the longitudinal extent of the elongate shape.

In this example, the second material contains the optical radiation within the second optical waveguide along the longitudinal extent of the elongate shape. The second material may be said to act as a cladding of the second optical waveguide. This simplifies the assembly of the optical measurement device. Moreover, it enables optical measurements to be made in a tissue region that is remote from the optical detector, e.g. in an MRI or X-ray imaging environment.

With reference to Fig. 1, in this example, the further portion 140'_{Pep} of the first optical waveguide may extend between the proximal end portion 140_{Pep} of the first optical waveguide and the distal end 140" of the first optical waveguide.

In another example, at least a portion of the second material 170 is formed in an elongate shape having a longitudinal extent, and wherein the at least a portion of the second material 170 is configured to contain the optical radiation within the second optical waveguide 150 along the longitudinal extent of the elongate shape.

In this example, the second material contains the optical radiation within the second optical waveguide along the longitudinal extent of the elongate shape. The second material may be said to act as a cladding of the second optical waveguide. This simplifies the assembly of the optical measurement device. Moreover, it enables optical measurements to be made in a tissue region that is remote from the optical detector, e.g. in an MRI or X-ray imaging environment.

With reference to Fig. 1, in this example, the further portion 150'_{Pep} of the second optical waveguide may extend between the proximal end portion 150_{Pep} of the second optical waveguide and the distal end 150" of the second optical waveguide.

In another example, the second material 170 comprises a tissue interface surface 170_{T} for interfacing with the tissue region 110. In this example, the further portion 140'_{Pep} of the first optical waveguide extends between the proximal end portion 140_{Pep} of the first optical waveguide and the distal end 140" of the first optical waveguide. The second material 170 comprises a tissue interface surface 170_{T} for interfacing with the tissue region 110. Furthermore, the distal end 140" of the first optical waveguide terminates at the tissue interface surface 170_{T} for irradiating the tissue region 110 via the distal end 140" of the first optical waveguide.

In this example, a tissue interface surface is provided by the second material. The second material therefore serves the dual purpose of interfacing with the tissue, as well as containing optical radiation within the first optical waveguide. This facilitates a more straightforward assembly of the optical measurement device.

In a related example, the tissue interface surface 170_{T} comprises at least one of: an absorbing region, a specular reflecting region, and a diffuse reflecting region. The absorbing region, or the specular reflecting region, or the diffuse reflecting region, is disposed between the distal end 140" of the first optical waveguide and the distal end 150" of the second optical waveguide. In this position, the absorbing region has the effect of reducing the opportunity for optical radiation to couple between the distal end of the first optical waveguide and the distal end 150" of the second optical waveguide without having passed through the tissue region. Such optical radiation might otherwise propagate between the distal end of the first optical waveguide and the distal end of the second optical waveguide via multiple reflections between the tissue interface surface 170_{T} and the surface of the tissue region. This optical radiation typically does not include useful information relating to biological effects within the tissue region, e.g. measurements of blood volume changes in the tissue region, and simply results in an offset in the electrical signal that is generated by the optical detector 130. In this position, the specular reflecting region, and the diffuse reflecting region, have the effect of re-cycling optical radiation that escapes from the tissue region, instead returning it to the tissue region, where it may acquire information relating to biological effects within the bulk of the tissue region, and ultimately propagate within the tissue region (e.g. via optical scattering) until it is incident on the distal end 150" of the second optical waveguide, and ultimately be detected by the optical detector. This improves the signal-to-noise ratio of the electrical signal that is generated by the optical detector. A combination of an absorbing region, and one or more specular/ diffuse reflecting regions may also be used. For instance, an absorbing region may be arranged in the form of a barrier between the distal end 140" of the first optical waveguide and the distal end 150" of the second optical waveguide, and the specular/ diffuse reflecting region(s) may be arranged around the distal end(s) of the first and/or second optical waveguide(s). This avoids the offset in the electrical signal that is generated by the optical detector, and also improves its signal-to-noise ratio. In these examples, the absorbing, or specular reflecting, or diffuse reflecting regions may be provided using techniques such as (dip-) coating, co-molding, or glueing.

This example is illustrated in Fig. 4, which is a schematic diagram illustrating a fourth example of an optical measurement device 400, in accordance with some aspects of the present disclosure. The tissue interface surface 170_{T} can be seen on the right-hand side of Fig. 4. The second material 170 in the optical measurement device 400 illustrated in Fig. 4 may be provided using a mold, for example.

In another example, the further portion 150'_{Pep} of the second optical waveguide extends between the proximal end portion 140_{PeP} of the second optical waveguide and the distal end 150" of the second optical waveguide. The second material 170 comprises a tissue interface surface 170_{T} for interfacing with the tissue region 110. Furthermore, the distal end 150" of the second optical waveguide terminates at the tissue interface surface 170_{T} for collecting the at least a portion of the optical radiation from the tissue region 110 via the distal end 150" of the second optical waveguide.

In this example, a tissue interface surface is provided by the second material. The second material therefore serves the dual purpose of interfacing with the tissue, as well as containing optical radiation within the second optical waveguide. This facilitates a more straightforward assembly of the optical measurement device.

In one example, a cross sectional area of the distal end 140" of the first optical waveguide is relatively larger than a cross sectional area of the proximal 140' end of the first optical waveguide. At least a portion of the first optical waveguide between the distal end 140" of the first optical waveguide and the proximal end 140' of the first optical waveguide comprises a tapered cross sectional area.

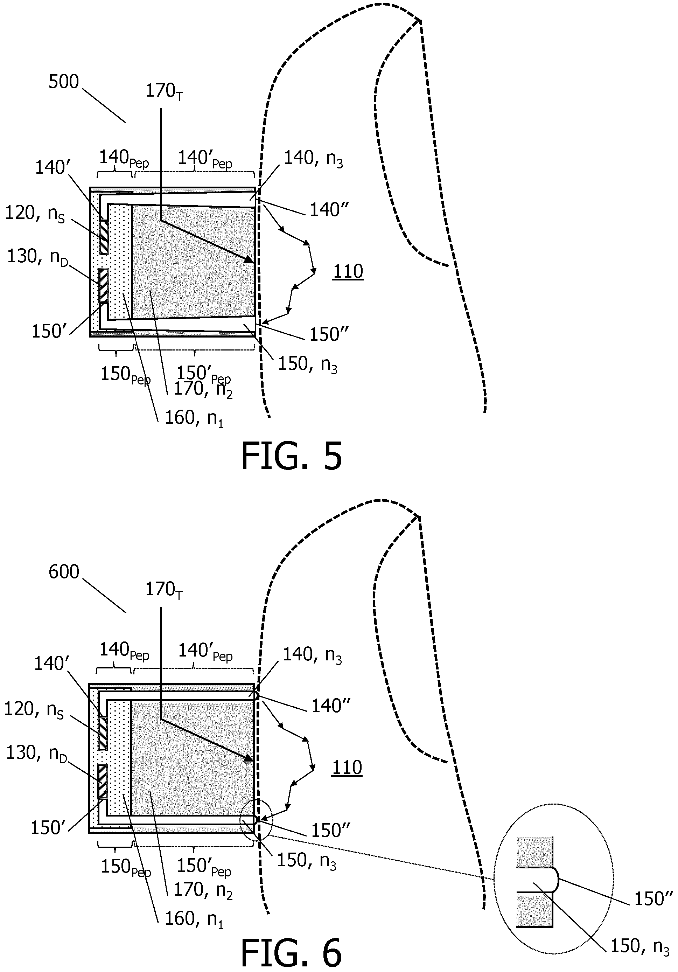

In this example, the tapered cross sectional area of the first optical waveguide increases the effective area of the optical source at the distal end of the first optical waveguide. This improves the optical coupling efficiency between the optical source and the tissue region. An example in which the first optical waveguide comprises a tapered cross sectional area is illustrated in Fig. 5, which is a schematic diagram illustrating a fifth example of an optical measurement device 500, in accordance with some aspects of the present disclosure. In this example, the further portion 140'_{Pep} of the first optical waveguide has a tapered cross sectional area. As compared to a waveguide with the same cross sectional area along its length, the tapered cross sectional area in this example has the effect of distributing the optical radiation that is emitted by the optical source 120 over a relatively larger surface area at the distal end 140" of the first optical waveguide. This in-turn improves the optical coupling efficiency between the optical source and the tissue region.

In one example, a cross sectional area of the distal end 150" of the second optical waveguide is relatively larger than a cross sectional area of the proximal end 150' of the second optical waveguide. At least a portion of the second optical waveguide between the distal end 150" of the second optical waveguide and the proximal end 150' of the second optical waveguide comprises a tapered cross sectional area.

In this example, the tapered cross sectional area of the second optical waveguide increases the effective area of the optical detector at the distal end of the second optical waveguide. This improves the optical collection efficiency of the optical radiation from the tissue region.

An example in which the second optical waveguide comprises a tapered cross sectional area is also illustrated in Fig. 5. In this example, the further portion 150'_{Pep} of the second optical waveguide has a tapered cross sectional area. As compared to a waveguide with the same cross sectional area along its length, the tapered cross sectional area in this example has the effect of collecting optical radiation from a relatively larger volume at the distal end 150" of the second optical waveguide. This in-turn improves the optical collection efficiency of the optical radiation from the tissue region.

In one example, the distal end 140" of the first optical waveguide and/or the distal end 150" of the second optical waveguide comprises a curved profile. The curved profile defines a convex lens.

In this example, the convex lens controls the spatial distribution of the optical radiation in the tissue region after it leaves the first optical waveguide and/or controls the volume of the tissue region from which optical radiation is collected by the second optical waveguide. Both, in-turn, improve the optical collection efficiency of the second optical waveguide. The convex lens may be provided in the form of a refractive lens or a diffractive lens.

An example in which the distal ends of the first and second optical waveguides 140, 150, comprise a curved profile, is illustrated in Fig. 6. Fig. 6 is a schematic diagram illustrating a sixth example of an optical measurement device 600, in accordance with some aspects of the present disclosure. The curved profile may be obtained using techniques such as polishing, and etching, for example. Diffractive optical elements may be obtained by molding, etching or printing.

As mentioned above, in one example, the optical source 120 and the optical detector 130 are provided by a photonic integrated circuit, "PIC". The optical source 120 and the optical detector 130 in the examples illustrated in Fig. 1 and in Fig. 3 - Fig. 6 may be provided by a PIC, for example. In a related example, the photonic integrated circuit 190 also includes at least a second optical source 120₂. The first optical waveguide 140 is further configured to couple optical radiation generated by the at least a second optical source 120₂, into the first optical waveguide 140.

In this example, the first optical waveguide is used to couple optical radiation from both optical sources to the tissue region. This provides a more straightforward assembly of the optical measurement device than e.g. using separate optical waveguides to couple optical radiation from the two sources to the tissue region.

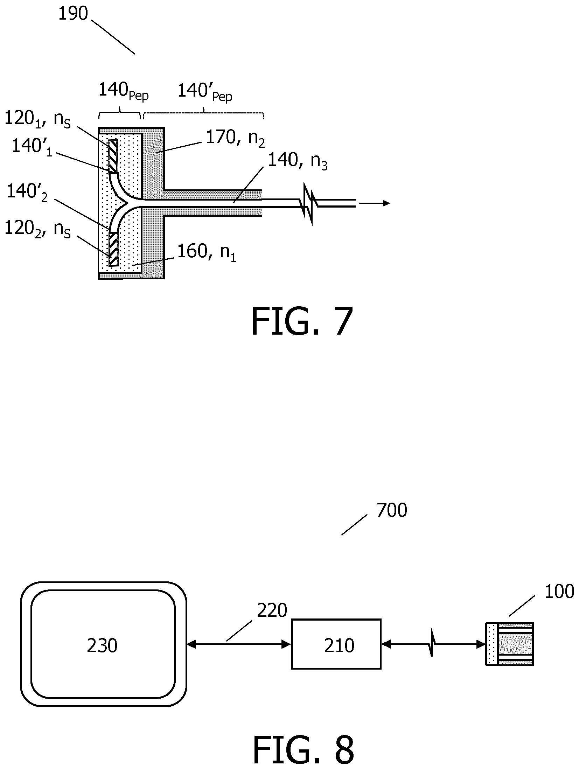

An example in which the first optical waveguide is used to couple optical radiation from two optical sources to a tissue region is illustrated in Fig. 7. Fig. 7 is a schematic diagram illustrating an example of a portion of a photonic integrated circuit 190 including two optical sources 120₁, and 120₂, in accordance with some aspects of the present disclosure. In the example illustrated in Fig. 7, the first optical waveguide 140 is split into two branches with corresponding distal ends 140'₁ and 140'₂. The distal ends 140'₁ and 140'₂ are butt-coupled to the optical source 120 and to the optical detector 130 respectively. The first optical waveguide 140 illustrated in Fig. 7 may extend to a tissue region 110 in the manner illustrated in Fig. 1 - Fig. 6. The branched first optical waveguide in this example may be provided by a spliced optical fiber, or by a strip waveguide in which the two branches are defined, for example. In one example, the first optical waveguide that is coupled to the optical sources, is also provided by the PIC.

As illustrated in Fig. 7, the first optical waveguide 140, and also the optical sources 120₁ and 120₂ of the PIC 190, are in direct contact with, i.e. touch, a first material 160 having a refractive index n₁. As described in relation to Fig. 1 - Fig. 7 above, the first material 160 contains optical radiation within the first optical waveguide and/or within the optical sources. The photonic integrated circuit 190 may also be provided with the optional second material 170. As described in the examples above, at least a portion of the first material 160, and also a further portion 140'_{Pep} of the first optical waveguide adjoining the proximal end portion 140_{PeP} of the first optical waveguide, are in direct contact with the second material 170. As described above, this has the effect of containing the optical radiation within the first optical waveguide 140.

The optical measurement devices described above may also be included in an optical measurement system. The optical measurement system may be used to obtain optical measurements from a tissue region 110. The optical measurement system includes:
the optical measurement device described in the examples above; and
a controller 210;

The controller is configured to:
generate control signals for causing the optical source 120 to generate the optical radiation;
receive electrical signals generated by the optical detector 130 in response to the detection of the at least a portion of the optical radiation;
evaluate a vital signs measurement 220 based on the received electrical signals; and
output the vital signs measurement 220.

In this example, the optical measurement system is used to evaluate a vital signs measurement. Thus, the optical measurement system may be used to assess the health of a subject.

Fig. 8 is a schematic diagram illustrating an example of an optical measurement system 700, in accordance with some aspects of the present disclosure. The optical measurement system 700 includes the controller 210 and an optical measurement device 100. The optical measurement system 700 may also include a display device 230 for displaying the vital signs measurement 220. The display device 230 may be any type of display device, such as a monitor, a virtual/ augmented reality display device, a display of a mobile communication device such as a mobile telephone, a tablet, and so forth. In the example illustrated in Fig. 8, the controller 210 is communication with the optical measurement device 100 and also with the display device 230. The communication may be effected any suitable form of data communication, including via wired, or wireless, or optical fiber communication.

In the example illustrated in Fig. 8, the controller 210 includes at least one processor. The at least one processor is programmed with instructions, which when executed by the processor, cause the processor to execute the operations of generating the control signals; receiving the electrical signals generated by the optical detector 130; and evaluating and outputting the vital signs measurement 220. The operations that are performed by the processor(s) may be stored on a computer-readable storage medium in the form of instructions which when executed by the one or more processors, cause the one or more processors to carry out the aforementioned operations.

The optical measurement system 700 may be used to evaluate various vital signs measurements. Examples of such various vital signs measurements include a PPG measurement, an SPG measurement, a blood oxygen saturation measurement, a heart rate measurement, a respiration rate measurement, an SpOz measurement, a temperature measurement, and so forth. Various known techniques may be used to derive these vital signs measurements from optical measurements in tissue. Fir instance, a blood oxygen saturation measurement may be obtained from PPG measurements in tissue using the ratio of pulse amplitudes of red and infrared PPG measurements (e.g. approximately 660 nm and approximately 880 nm or 940 nm, respectively) using the "ratio of ratios" technique. The vital signs measurement may be outputted to the display device 230 in various ways, including in the form of a number, a color code, and a trace representing the measurement over time.

It is noted that in addition to the controller 210 and the optical measurement device 100, the optical measurement system 700 illustrated in Fig. 8 may also include a user input device (not illustrated in Fig. 8) for receiving user input in relation to the operations performed by the controller 210, such as a touchscreen, a keypad, a keyboard, a mouse and so forth.

The above examples are to be understood as illustrative of the present disclosure, and not restrictive. Further examples are also contemplated. For instance, features described in relation to one optical measurement device, may be implemented in another optical measurement device, or in an optical measurement system, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. An optical measurement device (100, 200, 300, 400, 500, 600) for obtaining optical measurements from a tissue region (110), the optical measurement device comprising:
an optical source (120);
an optical detector (130);
a first optical waveguide (140); and
a second optical waveguide (150);
wherein the first optical waveguide (140) is configured to couple optical radiation generated by the optical source (120), from a proximal end (140') of the first optical waveguide to a distal end (140") of the first optical waveguide, for irradiating the tissue region (110) when the tissue region is in optical communication with the distal end (140") of the first optical waveguide (140);
wherein the second optical waveguide (150) is configured to couple at least a portion of the optical radiation collected from the tissue region (110) in response to the irradiating, from a distal end (150") of the second optical waveguide to the optical detector (130), via a proximal end (150') of the second optical waveguide;
wherein i) a proximal end portion (140_{Pep}) of the first optical waveguide adjoining the proximal end (140') of the first optical waveguide, and at least a portion of the optical source (120), are in direct contact with a first material (160) having a refractive index (n₁) that is less than a refractive index (n₃) of the first optical waveguide (140), and also less than a refractive index (n_{S}) of the optical source (120), for containing the optical radiation within the first optical waveguide (140) and/or within the optical source (120), respectively;
and/or
wherein ii) a proximal end portion (150_{Pep}) of the second optical waveguide adjoining the proximal end (150') of the second optical waveguide, and at least a portion of the optical detector (130), are in direct contact with a first material (160) having a refractive index (n₁) that is less than a refractive index (n₃) of the second optical waveguide (150), and also less than a refractive index (n_{D}) of the optical detector (130), for containing the optical radiation within the second optical waveguide (150) and/or within the optical detector (130), respectively.

2. The optical measurement device according to claim 1, wherein i) a proximal end portion (140_{Pep}) of the first optical waveguide adjoining the proximal end (140') of the first optical waveguide, and at least a portion of the optical source (120), are in direct contact with a first material (160) having a refractive index (n₁) that is less than a refractive index (n₃) of the first optical waveguide (140), and also less than a refractive index (n_{S}) of the optical source (120), for containing the optical radiation within the first optical waveguide (140) and/or within the optical source (120), respectively; and
wherein at least a portion of the first material (160), and a further portion (140'_{Pep}) of the first optical waveguide adjoining the proximal end portion (140_{Pep}) of the first optical waveguide, are in direct contact with a second material (170) having a refractive index (n₂) that is less than a refractive index (n₃) of the first optical waveguide (140) for containing the optical radiation within the first optical waveguide (140).

3. The optical measurement device according to claim 1 or claim 2, wherein ii) a proximal end portion (150_{Pep}) of the second optical waveguide adjoining the proximal end (150') of the second optical waveguide, and at least a portion of the optical detector (130), are in direct contact with a first material (160) having a refractive index (n₁) that is less than a refractive index (n₃) of the second optical waveguide (150), and also less than a refractive index (n_{D}) of the optical detector (130), for containing the optical radiation within the second optical waveguide (150) and/or within the optical detector (130), respectively; and
wherein at least a portion of the first material (160), and a further portion (150'_{Pep}) of the second optical waveguide adjoining the proximal end portion (150_{Pep}) of the second optical waveguide, are in direct contact with a second material (170) having a refractive index (n₂) that is less than a refractive index (n₃) of the second optical waveguide (150) for containing the optical radiation within the second optical waveguide (150).

4. The optical measurement device (100, 200) according to claim 2, wherein at least a portion of the second material (170) is formed in an elongate shape having a longitudinal extent, and wherein the at least a portion of the second material (170) is configured to contain the optical radiation within the first optical waveguide (140) along the longitudinal extent of the elongate shape.

5. The optical measurement device (100, 200) according to claim 3, wherein at least a portion of the second material (170) is formed in an elongate shape having a longitudinal extent, and wherein the at least a portion of the second material (170) is configured to contain the optical radiation within the second optical waveguide (150) along the longitudinal extent of the elongate shape.

6. The optical measurement device (400, 500, 600) according to any one of claims 2-5, wherein the further portion (140'_{Pep}) of the first optical waveguide extends between the proximal end portion (140_{Pep}) of the first optical waveguide and the distal end (140") of the first optical waveguide, and wherein the second material (170) comprises a tissue interface surface (170_{T}) for interfacing with the tissue region (110); and
wherein the distal end (140") of the first optical waveguide terminates at the tissue interface surface (170_{T}) for irradiating the tissue region (110) via the distal end (140") of the first optical waveguide.

7. The optical measurement device (400, 500, 600) according to any one of claims 2-6, wherein the further portion (150'_{Pep}) of the second optical waveguide extends between the proximal end portion (140_{Pep}) of the second optical waveguide and the distal end (150") of the second optical waveguide, and wherein the second material (170) comprises a tissue interface surface (170_{T}) for interfacing with the tissue region (110); and
wherein the distal end (150") of the second optical waveguide terminates at the tissue interface surface (170_{T}) for collecting the at least a portion of the optical radiation from the tissue region (110) via the distal end (150") of the second optical waveguide.

8. The optical measurement device (500) according to claim 6 or claim 7, wherein a cross sectional area of the distal end (140") of the first optical waveguide is relatively larger than a cross sectional area of the proximal (140') end of the first optical waveguide and wherein at least a portion of the first optical waveguide between the distal end (140") of the first optical waveguide and the proximal end (140') of the first optical waveguide comprises a tapered cross sectional area;
and/or
wherein a cross sectional area of the distal end (150") of the second optical waveguide is relatively larger than a cross sectional area of the proximal end (150') of the second optical waveguide and wherein at least a portion of the second optical waveguide between the distal end (150") of the second optical waveguide and the proximal end (150') of the second optical waveguide comprises a tapered cross sectional area.

9. The optical measurement device (600) according to any one of claims 6-8, wherein the distal end (140") of the first optical waveguide and/or the distal end (150") of the second optical waveguide comprises a curved profile, and wherein the curved profile defines a convex lens.

10. The optical measurement device (100, 200) according to any previous claim, wherein the distal end (140") of the first optical waveguide and the distal end (150") of the second optical waveguide are arranged in a side-by-side configuration for obtaining a reflective optical measurement from the tissue region (110);
or
wherein the distal end (140") of the first optical waveguide and the distal end (150") of the second optical waveguide are arranged in an opposing configuration for obtaining a transmissive optical measurement from the tissue region (110).

11. The optical measurement device according to any previous claim, wherein the first material (160) comprises a rigid material and/or wherein the second material (170) comprises a rigid or flexible material.

12. The optical measurement device according to any previous claim, wherein the first material (160) and/or the second material (170) comprises at least one of: a transparent material, an opaque material, a scattering material, and an absorbing material.

13. The optical measurement device according to any previous claim, wherein the optical source (120) and the optical detector (130) are provided by a photonic integrated circuit.

14. The optical measurement device according to claim 13, wherein the photonic integrated circuit (190) further comprises at least a second optical source (120₂); and
wherein the first optical waveguide (140) is further configured to couple optical radiation generated by the at least a second optical source (120₂), into the first optical waveguide (140).

15. An optical measurement system for obtaining optical measurements from a tissue region (110), the optical measurement system comprising:
the optical measurement device according to any previous claim; and
a controller (210);
wherein the controller is configured to:
generate control signals for causing the optical source (120) to generate the optical radiation; receive electrical signals generated by the optical detector (130) in response to the detection of the at least a portion of the optical radiation;
evaluate a vital signs measurement (220) based on the received electrical signals; and output the vital signs measurement (220).
